# EUROPEAN PATENT APPLICATION

(11) **EP 1 029 846 A1**
(43) Date of publication of application: **23.08.2000**
(21) Application number: 99301161.8
(22) Date of filing: 17.02.1999
(51) Int. Cl.: C07C 51/487, C07C 59/64, C07C 59/84, C07C 57/30, C07C 57/58, C07B 57/00

(54) **Resolution of alpha-arylpropionic acids**

(71) Applicant: Sekhsaria Chemicals Ltd., Mumbai 400 021 (IN)
(72) Inventor: Oak, Ganesh M., Naupada, Thane (W) 400 602 (IN); Chodankar, Nandkumar K., Juhu, Mumbai (IN)
(74) Representative: Curtis, Philip Anthony

(57) **Abstract**

A method of resolving d- and I-isomers of an α-arylpropionic acid, wherein the isomers are enantiomers. The method involves forming an ammonium salt of the α-arylpropionic acid, then dissolving in a solvent the ammonium salt, and a chiral agent capable of reacting with the ammonium salt of at least one of the isomers. The reaction product of the chiral agent and the ammonium salt of the one of the isomers can subsequently be purified, then separated into the chiral agent and the isomer.

## Description

This invention relates to the resolution of α-arylpropionic acids.

α-arylpropionic acids are well known as non-steroidal antiinflammatory (NSAI) agents, and include compounds such as naproxen, ibuprofen and ketoprofen. These compounds are chiral, and the routes used to synthesise them result in the formation of a mixture containing d- and I-enantiomers. However, the active compound usually comprises only one of the enantiomers of the racemic mixture.

Naproxen is 2-(6-methoxy-2-naphthyl) propionic acid. When naproxen is synthesised, it is formed as a mixture of d-naproxen and I-naproxen, which will be indicated as dl-naproxen throughout this specification. However, it is the d-naproxen that is pharmaceutically active. As a result, there has been a great deal of interest in finding methods of resolving dl-naproxen, so that the d-naproxen can be separated from the I-naproxen.

South African patent no. 700809 describes a number of resolving agents for the resolution of naproxen. Most of these resolving agents are expensive and/or ecologically unfriendly.

GB-A-2025968 describes the use of an organic base, such as triethylamine, and inorganic bases, such as sodium/potassium hydroxide, for preparing salts with dl-naproxen. The salts are then resolved using N-methyl-D-glucamine or similar resolving agents.

US-A-5235100 also describes the use of an organic base, such as triethylamine, to form a salt, which is then resolved using (-)-α-phenylethylamine without using a solvent.

EP-A-0298395 describes the formation of a monoethylamine salt of dl-naproxen. Resolution is carried out by forming a supersaturated solution of this salt of dl-naproxen in a solvent such as aqueous acetone, then seeding the solution with one of the enantiomers of naproxen.

Indian patent no. 163,260 describes the resolution of the potassium salt of dl-naproxen using (-)-a-phenylethylamine in water.

In WO-A-93/15040 there is described a method of preparing optically active carboxylic acids. This specification discloses a method for separating the enantiomers of a racemic mixture of a C₁ to C₆ linear or branched aliphatic carboxylic acid or ester thereof, the process comprising: (i) forming a salt solution of said racemic mixture and an organic or inorganic base; (ii) treating said salt solution with a chiral organic nitrogenous base having a base strength no stronger than said organic or inorganic base; and (iii) precipitating from the reaction solution formed in step (ii) the less soluble diastereomeric salt; and (iv) separating the precipitated less soluble diastereomeric salt. This method is also disclosed in US patent no. 5015764. The improvement claimed in WO-A-93/15040 comprises adding to the salt solution of step (i) an organic or inorganic salt soluble in the salt solution. The organic or inorganic salt may be an alkali metal salt, an alkaline earth metal salt or an ammonium salt: sodium chloride is especially preferred. Furthermore, it is essential that the organic or inorganic salt is inert to the rest of the reactants.

We have now found another way of resolving optically active acids, particularly α-arylpropionic acids such as dl-naproxen, which involves forming an ammonium salt of the d- and I-isomers of the acid, then reacting the ammonium salt with a chiral agent in a solvent. The invention is based upon the discovery that the ammonium salt of the acid can react with the chiral agent to produce diastereomers having significantly different solubilities in the solvent. This makes it possible to separate the d- and I-isomers much more easily than has been possible in the prior art.

According to the present invention there is provided a method of resolving d- and I-isomers of an α-arylpropionic acid, wherein said isomers are enantiomers, comprising forming an ammonium salt of the d- and I-isomers of the α-arylpropionic acid, and dissolving the ammonium salt and a chiral agent in a solvent, the chiral agent being capable of reacting with the ammonium salt of at least one of the d- and I-isomers.

The chiral agent is preferably an organic nitrogenous material, and is more preferably an amine. It is desirable that the chiral agent is a chiral base, or a hydrochloride of a chiral base. The chiral agent is preferably a phenylalkylamine or a hydrochloride of a phenylalkylamine. The alkyl group preferably contains two or three carbon atoms, such as α-phenylpropylamine and α-phenylethylamine. Most preferably the alkyl group contains two carbon atoms.

The most preferred chiral agent is either (-)-α-phenylethylamine or (-)-α-phenylethylamine hydrochloride for resolution of d-α-arylpropionic acids such as d-Naproxen, and (+)-α-phenylethylamine or (+)-α-phenylethylamine hydrochloride for resolution of R(-)α-arylpropionic acids such as R(-) Naproxen.

It is possible for the phenyl group to contain an alkyl group substituent, particularly a methyl substituent; an example of such a chiral agent is α-(4-methylphenyl)ethylamine. In addition, or instead, the phenylalkylamine may contain two phenyl groups; an example of such a chiral agent is 1-phenyl-2-(4-methylphenyl)ethylamine.

There are a wide variety of solvents that may be used. The solvent should be capable of dissolving the ammonium salt of the d- and I-isomers. We prefer to use water as the solvent, but other possibilities include an aqueous alcohol, like methanol, isopropanol and butanol, or chloroform.

It is preferred that the ammonium salt is formed by reacting the α-arylpropionic acid with aqueous ammonia, with ammonia gas, or with ammonia dissolved in an aqueous alcohol. The step of forming the ammonium salt of the α-arylpropionic acid results in the formation of the ammonium salt of each of the d-and I-isomers. It is a specific feature of the invention that the ammonium salt is formed by reacting the ammonia or a suitable ammonium compound with the α-arylpropionic acid; thus, the ammonia or ammonium compound is not inert to the α-arylpropionic acid. It will be appreciated that there is no need to react the α-arylpropionic acid with any other material prior to forming the ammonium salt.

In one conventional process for manufacturing dl-naproxen, the corresponding α-arylproprionitrile is hydrolysed to form crude dl-naproxen which is then purified by conversion to its ammonium salt. It is a feature of the invention to use the ammonium salt formed from the hydrolysed α-arylproprionitrile for the resolution.

The ammonium salt and said chiral agent are preferably dissolved in the solvent at a temperature in the range 80°C to 95°C. The ammonium salt can be dissolved in the solvent before, after, or at the same time as, the chiral agent is dissolved in the solvent.

It has been found that the relative amount of the chiral agent and the α-arylpropionic acid is important. It is preferred that the amount of the chiral agent, in mole equivalent, comprises 45% to 60% of the amount of the ammonium salt. It is particularly preferred that the amount of the chiral agent, in mole equivalent, comprises about 50% of the amount of the ammonium salt.

In an embodiment of the invention it is possible to dissolve an amount of said α-arylpropionic acid in said solvent with said ammonium salt and said chiral agent. In this embodiment, the amount of the α-arylpropionic acid, in mole equivalent, is preferably about equal to the amount of said ammonium salt. The amount of the chiral base, in mole equivalent, preferably comprises 45% to 60%, more preferably about 50%, of the combined amount of the α-arylpropionic acid and the ammonium salt.

It will be clear from the foregoing that the chiral agent should be a material that is capable of reacting with the ammonium salt of at least the d- or I-isomer of the α-arylpropionic acid. In the preferred embodiment, the ammonium salt of both the d- and I-isomers will form reaction products with the chiral agent; in this embodiment, the reaction products will be diastereomers having different solubilities in the solvent, and will therefore be separable by a physical separation process, such as precipitation, followed by filtration. In another embodiment, only the ammonium salt of the d-isomer will form a reaction product with the chiral agent; in this embodiment the reaction product will be separable from the ammonium salt of I-isomer by a physical separation process, such as precipitation followed by filtration. In a still further embodiment, only the ammonium salt of the I-isomer will form a reaction product with the chiral agent; in this embodiment the reaction product will be separable from the ammonium salt of d-isomer by a physical separation process, such as precipitation followed by filtration.

Thus, after the chiral agent and the ammonium salt have been added to the solvent, it will be usual to separate the reaction product of the ammonium salt of one of the d- and I-isomers either from the reaction product of the ammonium salt of the other of the d- and I-isomers, or from the ammonium salt of the other of the d- and I-isomers. This separation is preferably carried out by precipitation, followed by filtration.

The residue of the filtration may be purified by crystallisation, preferably by crystallisation from an aqueous organic solvent, such as an aqueous alcohol; aqueous methanol is particularly suitable as a solvent. The purified residue can be treated in order to convert the reaction product of the ammonium salt of the d- or I-isomer into a mixture of the d- or I-isomer and the chiral agent.

In one embodiment, the treatment involves the addition of an alkali, such as an alkali metal hydroxide, especially sodium hydroxide. The chiral agent may be separated from the alkaline solution by liquid-liquid extraction using a solvent such as toluene; several stages of liquid-liquid extraction may be used. The remaining alkaline solution may be acidified, for example with hydrochloric acid, to yield the d- or I-isomer in a substantially pure form. It is possible that the d- or I-isomer thus formed does not have the required degree of purity, i.e., there may be an undesirably large amount of the other isomer. In this case, it is possible to repeat the process, in order to further purify the d- or I-isomer.

In another embodiment, the treatment involves the addition of an acid, such as hydrochloric acid. In this embodiment, the addition of the acid results in the formation of the d- or I-isomer of the α-arylpropionic acid and the hydrochloride of the chiral agent.

The filtrate will contain either the reaction product of the ammonium salt of the other of the d- and I-isomer, or the ammonium salt of the other of the d-and I-isomer, which can be racemised directly. The reaction product may be treated with an acid or alkali, as described above, which will cause the precipitation of the other of the d- or I-isomers. The precipitated d- or I-isomer may be racemised by the addition of an alkali, such as an alkali metal hydroxide, especially potassium hydroxide. This step is desirably carried out during the hydrolysis of α-arylproprionitrile to dl-naproxen: the other of the d- and I-isomers can be added to the α-arylproprionitrile, and potassium hydroxide can be added, for example, to hydrolyse the α-arylproprionitrile and to racemise the other of the d- and I-isomers.

The chiral agent recovered from the process may be purified, then recycled to the start of the process.

The most preferred embodiment of the present invention involves the following steps:
(1) formation of the ammonium salt of dl-α-arylpropionic acid;
(2) formation of the (-)-α-phenylethylamine salt of d-α-arylpropionic acid and of l-α-arylpropionic acid;
(3) purification of the (-)-α-phenylethylamine salt of d-α-arylpropionic acid;
(4) breaking the (-)-α-phenylethylamine salt of d-α-arylpropionic acid into d-α-arylpropionic acid and (-)-α-phenylethylamine;
(5) separating the d-α-arylpropionic acid from the (-)-α-phenylethylamine;
(6) breaking the (-)-α-phenylethylamine salt of l-α-arylpropionic acid into l-α-arylpropionic acid and (-)-α-phenylethylamine;
(7) racemisation of the l-α-arylpropionic acid to dl-α-arylpropionic acid.

The invention can be used with a wide variety of α-arylpropionic acids, including naproxen, ibuprofen, ketoprofen, flurbiprofen and 2-(4-methylphenyl) propionic acid. The α-aryl propionic acids have the following general structure: 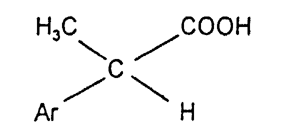 where Ar represents a group containing at least one phenyl or naphthyl group. The structure of specific acids which can be resolved in accordance with the present invention are as follows:

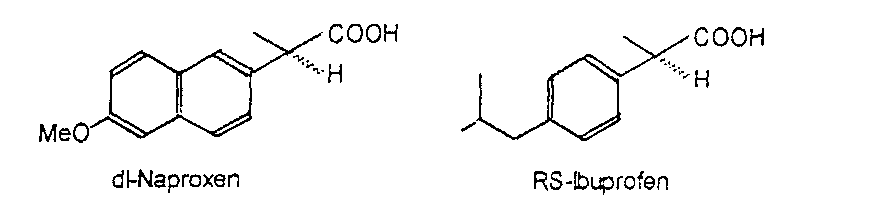

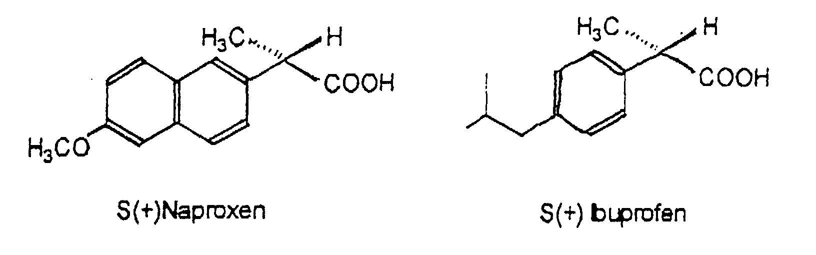

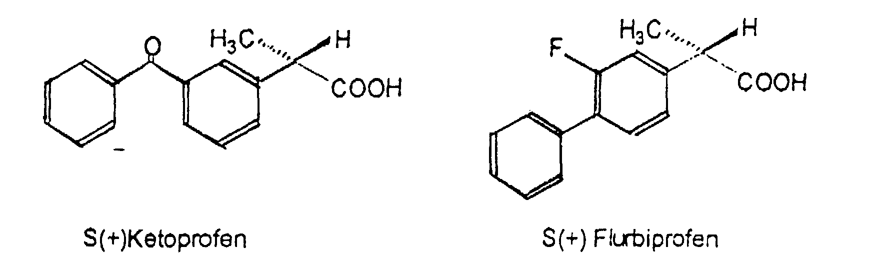

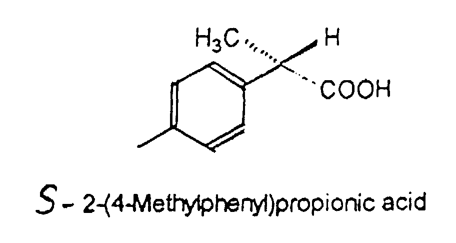

The method according to the invention has a number of advantages over the prior art. Previous attempts to resolve, for example, dl-naproxen have required several purification steps in order to carry out the resolution effectively. With the present invention it is possible to obtain d-naproxen of good purity with only a single purification step.

Furthermore, with the present invention it is necessary to use only 0.5 molar equivalent of the chiral agent, and the solvent for the chiral agent and the dl-naproxen may be water.

Moreover, the ammonium salt required for the process of the present invention can be prepared in the course of the conventional manufacturing process for dl-naproxen. This has the effect that fewer process steps are required to obtain the d-naproxen.

The invention may be used to produce either of the d- and I-isomers of the α-arylproprionic acid in substantially pure form. Thus, for example, the invention may be used to produce d-naproxen or I-naproxen.

It will of course be appreciated that the invention may be applied to the separation of certain derivatives of a-arylpropionic acids, such as the esters thereof.

The following examples illustrate the invention.

### Example 1

The following method was used to resolve dl-naproxen into d-naproxen:
(A) 350 g (1.5217 moles) dl-naproxen was converted into its ammonium salt by treating it with aqueous ammonia solution/ammonia gas. The resulting ammonium salt was then suspended in 4-8 volumes water. To the suspension, 350 g dl-naproxen was added. The slurry was heated to 80-85°C under a nitrogen blanket.
(B) To the slurry produced in step (A), 184 g (1.5206 moles) (-)-α-phenylethylamine ("PEA") was added, over a period of 30-120 minutes at a temperature of 80-95°C. The reaction mixture was held at 80-95 °C for 1-3 hours, then filtered to obtain 340-360 g of a crude d-naproxen PEA salt (melting range 160-170°C α_{D}>+30° CH₃CN-HOAc) having an optical purity above 70%.
(C) The crude salt from step (B) was then crystallised from 4 volumes of aqueous methanol to yield 250-270 g pure d-naproxen PEA salt (melting range 170-175°C, α_{D}≥+37° CH₃CN-HOAc) having an optical purity above 95%.
(D) The pure d-naproxen PEA salt from step (C) was then reacted with dilute aqueous hydrochloric acid. PEA remained in the aqueous solution as hydrochloride. 165-175 g of d-naproxen was precipitated having a melting point of 156-158°C and α_{D} CHCI₃≥+63.5° CHCI₃.
(E) The aqueous filtrate and washings from step (B) were cooled to 0-10°C to isolate a mixture of ammonium salt of dl-naproxen, and I-naproxen PEA salt. This salt mixture was then treated with dilute hydrochloric acid, whereupon naproxen enriched in I-isomer precipitated out and PEA remained in aqueous solution as hydrochloride.
(F) The precipitated I-naproxen from step (E) was racemised with potassium hydroxide to obtain 500-520 g of dl-naproxen.
(G) The dl-naproxen obtained from step (F) and the PEA recovered from steps (D) and (E) were recycled to step (A), along with fresh dl-naproxen.

### Example 2

The following method was used to resolve dl-naproxen into d-naproxen:
(A) 700 g (3.04034 moles) dl-naproxen was converted into its ammonium salt by treating it with aqueous ammonia solution/ammonia gas. The resulting ammonium salt was then suspended in 5-7 volumes water. The slurry was heated to 80-85°C under a nitrogen blanket.
(B) To the slurry produced in step (A), PEA hydrochloride equivalent to 184 g (1.5206 moles) PEA was added over a period of 30-120 minutes at a temperature of 80-95°C. The reaction mixture was held at 85-95°C for 1-3 hours, then filtered to obtain 340-360 g of a crude d-naproxen PEA salt (melting range 160-170°C, α_{D}>+30° CH₃CN-HOAc) having an optical purity above 70%.
(C) The crude salt from step (B) was then crystallised from 4 volumes of aqueous methanol to yield 250-270 g pure d-naproxen PEA salt (melting range 170-175°C, α_{D}≥+37° CH₃CN-HOAc) having an optical purity above 95%.
(D) The pure d-naproxen PEA salt from step (C) was then reacted with dilute aqueous hydrochloric acid. PEA remained in the aqueous solution as hydrochloride, 165-175 g of d-naproxen was precipitated having a melting point of 156-158°C and α_{D} CHCI₃≥+63.5° CHCI₃.
(E) The aqueous filtrate and washings from step (B) were cooled to 0-10°C to isolate a mixture of ammonium salt of dl-naproxen, and I-naproxen PEA salt. This salt mixture was then treated with dilute aqueous hydrochloric acid, whereupon naproxen enriched in I-isomer precipitated out and PEA remained in aqueous solution as hydrochloride.
(F) The precipitated I-naproxen from step (E) was racemised with potassium hydroxide to obtain 500-520 g of dl-naproxen.
(G) The dl-naproxen obtained from step (F) and the PEA recovered from steps (D) and (E) were recycled to step (A), along with fresh dl-naproxen.

### Example 3

The following method was used to resolve dl-naproxen into d-naproxen:
(A) Steps (A) and (B) of Example 1 were repeated to obtain 340-360 g of the crude d-naproxen PEA salt (alternatively steps (A) and (B) of Example 2 could have been repeated instead).
(B) The crude d-naproxen PEA salt was crystallised from aqueous methanol to obtain 250-270 g of pure d-naproxen PEA salt.
(C) The pure salt was then treated with a dilute aqueous solution of potassium hydroxide at 10-30°C. This resulted in the formation of PEA and an aqueous solution of potassium salt of d-naproxen. The PEA was separated by extraction with toluene. The potassium solution of d-naproxen was acidified, filtered, then dried, yielding 150-170 g of d-naproxen having a melting point of 156-158°C and α_{D} CHCI₃≥+63.5° CHCI₃.
(D) The toluene solution containing the PEA was treated with aqueous hydrochloric acid to separate PEA hydrochloride, which was recycled. The toluene was washed, then recycled.
(E) Steps (E), (F) and (G) of Example 1 (or, alternatively, Example 2), were repeated.

It will be appreciated that modifications are possible to the invention described above.

## Claims

**1.** A method of resolving d- and I-isomers of an α-arylpropionic acid, wherein said isomers are enantiomers, comprising forming an ammonium salt of the d- and I-isomers of the α-arylpropionic acid, and dissolving the ammonium salt and a chiral agent in a solvent, the chiral agent being capable of reacting with the ammonium salt of at least one of the isomers.

**2.** A method according to claim 1, wherein the chiral agent is an organic nitrogenous material.

**3.** A method according to claim 1 or 2, wherein the chiral agent is (-)-α-phenylethylamine or (-)-α-phenylethylamine hydrochloride or (+)-α-phenylethylamine or (+)-α-phenylethylamine hydrochloride.

**4.** A method according to claim 1, 2 or 3, wherein said ammonium salt is formed by reacting said α-arylpropionic acid with aqueous ammonia, ammonia gas, or ammonia dissolved in an aqueous alcohol.

**4.** A method according to any preceding claim, wherein said ammonium salt and said chiral agent are dissolved in the solvent at a temperature in the range 80°C to 95°C.

**5.** A method according to any preceding claim, wherein the amount of the chiral agent, in mole equivalent, comprises 45% to 60% of the amount of the ammonium salt.

**6.** A method according to any one of claims 1 to 4, further comprising dissolving an amount of said α-arylpropionic acid in said solvent with said ammonium salt and said chiral agent.

**7.** A method according to claim 6, wherein the amount of the α-arylpropionic acid, in mole equivalent, is about equal to the amount of said ammonium salt.

**8.** A method according to claim 7, wherein the amount of the chiral base, in mole equivalent, comprises 45% to 60% of the combined amount of the α-arylpropionic acid and the ammonium salt.

**9.** A method according to any preceding claim, further comprising at least partially separating the reaction product of the chiral base and the ammonium salt of one of the d- and I-isomers, from the other of the d- and I-isomers and/or from the reaction product of the chiral base and the ammonium salt of the other of the d- and I-isomers.

**10.** A method according to claim 9, further comprising recovering said one of the d- and I- isomers from said reaction product.

**11.** A method according to claim 10, comprising:
(a) treating said reaction product with an acid to produce said one of the d- and I-isomers, and recovering said one of the d- and I-isomers in a substantially pure form; or
(b) treating said reaction product with an alkali metal hydroxide to produce an alkali metal salt of said one of the d- and I-isomers, recovering said alkali metal salt, acidifying the recovered alkali metal salt to produce said one of the d- and I-isomers, and recovering said one of the d- and I-isomers in a substantially pure form.

**12.** A method according to claim 10 or 11, comprising recovering said chiral agent from the reaction product of the chiral agent and said one of the d- and I-isomers, then recycling said recovered chiral agent for dissolution in the solvent.

**13.** A method according to claim 10, 11 or 12, wherein said other of the d- and I-isomers is racemised to form at least part of the α-arylpropionic acid dissolved in the solvent.

**14.** A method according to any preceding claim, comprising forming said α-arylpropionic acid by hydrolysing a corresponding α-arylproprionitrile, and then forming said ammonium salt directly from the α-arylpropionic acid formed from said hydrolysis.

**15.** A method according to claim 14, when dependent upon claim 13, wherein said other of the d- and I-isomers is racemised by adding it to the α-arylproprionitrile, and hydrolysing the mixture with an alkali metal hydroxide.
